# EUROPEAN PATENT APPLICATION

(11) **EP 1 895 304 A1**
(43) Date of publication of application: **05.03.2008**
(21) Application number: 06018277.1
(22) Date of filing: 31.08.2006
(51) Int. Cl.: G01N 33/68, C12Q 1/68

(54) **Methods for detecting protein-peptide interactions**

(71) Applicant: Stiftung caesar Center of Advanced European Studies and Research, 53175 Bonn (DE)
(72) Inventor: Hennemann, Hanjo, 41470 Neuss (DE); Kruse, Claudia, 53115 Bonn (DE); Hanke, Sabine, 53809 Ruppichteroth-Winterscheid (DE); Friebe, Annette, 51399 Burscheid (DE)
(74) Representative: Müller Fottner Steinecke

(57) **Abstract**

A novel method of detecting and characterizing protein-peptide interactions is provided, which can be used for isolating peptides and proteins, respectively, as well as in drug screening and development.

SEQUENCE LISTING

<110> Stiftung caesar

<120> Means and methods for detecting protein-peptide interactions

<130> CA18A04/P-EP

<160> 10

<170> PatentIn version 3.3

<210> 1
<211> 24
<212> PRT
<213> artificial sequence

<220>
<223> oligopeptide

<400> 1

Gly Pro Gly Asp Thr Tyr Arg Pro Lys Arg Pro Thr Thr Leu Asn Leu
1               5                   10                  15

Phe Pro Gln Val Pro Arg Ser Gly
            20

<210> 2
<211> 69
<212> DNA
<213> artificial sequence

<220>
<223> oligonucleotide

<220>
<221> misc_feature

<222>  (1)..(69)
<223>  Primer (JIP20fRSR115)

<400>  2
gtccggggga cacgtaccgg cccaagcggc ccaccacgct caacctcttt ccgcaggtgc      60

cgcggagcg                                                              69

<210>  3
<211>  69
<212>  DNA
<213>  artificial sequence

<220>
<223>  oligonucleotide

<220>
<221>  misc_feature
<222>  (1)..(69)
<223>  Primer (JIP20rRSRII)

<400>  3
gaccgctccg cggcacctgc ggaaagaggt tgagcgtggt gggccgcttg ggccggtacg      60

tgtcccccg                                                              69

<210>  4
<211>  15
<212>  PRT
<213>  artificial sequence

<220>
<223>  oligopeptide

<400>  4

Thr Tyr Arg Pro Lys Arg Pro Thr Thr Leu Asn Leu Phe Pro Gln
1               5                   10                  15

<210>  5
<211>  15
<212>  PRT
<213>  artificial sequence

<220>
<223>  oligopeptide

<400>  5

Gln Pro Phe Leu Asn Leu Thr Thr Pro Arg Lys Pro Arg Tyr Thr
1               5                   10                  15

<210>  6
<211>  15
<212>  PRT
<213>  artificial sequence

<220>
<223>  oligopeptide

<400>  6

Leu Arg Phe Gln Pro Tyr Pro Lys Asn Leu Thr Pro Thr Arg Thr
1               5                   10                  15

<210>  7
<211>  31
<212>  DNA
<213>  artificial sequence

<220>
<223>  oligonucleotide

<220>
<221>  misc_feature

<222>  (1)..(31)
<223>  Primer (P1trxASmaIforw)

<400>  7
cccccgggga tgagcgataa aattattcac c                                    31

<210>  8
<211>  29
<212>  DNA
<213>  artificial sequence

<220>
<223>  oligonucleotide

<220>
<221>  misc_feature
<222>  (1)..(29)
<223>  Primer (P2trxAEcoRIrev)

<400>  8
ttttgaattc ccgccaggtt agcgtcgag                                       29

<210>  9
<211>  31
<212>  DNA
<213>  artificial sequence

<220>
<223>  oligonucleotide

<220>
<221>  misc_feature
<222>  (1)..(31)
<223>  Primer (P3trxASmaIforw)

<400>  9
cccccgggga tgagcgataa aattattcac c                                    31

<210>  10

<211>  36

<212>  DNA

<213>  artificial sequence

<220>

<223>  oligonucleotide

<220>

<221>  misc_feature

<222>  (1)..(36)

<223>  Primer (P4JipXho)

<400>  10

tcgagtcact gcggaaagag gttgagcgtg gtgggc                                36

## Description

### Field of the invention

The present invention generally relates to the technical field of molecular biology. More specifically, the invention relates to method for detecting and identifying peptides involved in a protein-peptide interaction in cellular systems. In particular, the present invention relates to methods for detecting, identifying and optionally isolating peptides such as functional peptide ligands of target proteins.

### Background of the invention

Peptides show a wide variety of biological activities in cells one important example of which are natural peptide hormones acting as important molecular signals by binding to cellular receptors. It has also been shown that synthetic peptides can modulate the activity of proteins and even inhibit protein-protein interactions and are therefore important lead compounds in the field of drug discovery. Due to their high activity and specificity, during the last years there was an increased focus on peptides as therapeutic effectors.

The development of methods for detecting interactions between target proteins and peptides under physiological conditions is of major interest. Although there exist already a number of methods for detecting interactions between proteins and peptide ligands as well as for isolating novel peptide ligands, in particular, methods for identifying novel peptide ligands are of special interest in drug discovery. However, existing *in vitro* methods for the identification of binding peptides (Liu et al., Exp. Hematology 31 (2003), 11-30) require as a prerequisite the purification of the protein of interest, resulting in most cases in a decreased biological activity and binding quality, since many target proteins adopt their preferred conformation exclusively in the cytoplasm which can differ from their conformation under non-physiological conditions.

One common method for isolating peptide ligands allowing the presentation of peptide libraries on the surface of a filamentous phage (Smith, Science 228 (1985), 1315-1317), comprises the introduction of exogenous peptide sequences into the genome of phage capsid proteins, because of which this method is also referred to as phage display technology. International application WO95/31723 describes the use of this method for *in vitro* selection of peptides from biological peptide libraries, but although there are few examples for its application to living organisms (Arap et al., Proc. Natl. Am. Soc. 99 (2002), 1527-1531), its use for *in vivo* selection is limited.

To date, there exist different methods for identifying binding partners in cellular systems. In this context, yeast as a eukaryotic cell is a preferred organism to analyse protein-protein interaction *in vivo.* One characteristic method using yeast as a preferred organism is the yeast two-hybrid system, which represents a further development of the two-hybrid protein interaction assay (Fields and Song, Nature 349 (1989), 245-246). These cellular methods comprise one binding partner fused to a DNA binding domain, while the respective other binding partner is fused to a strong transcription activation domain, and as a result of the molecular interaction of the partners the generation of a functional transcription factor, leading to a change in phenotype of the cell due to expression of respective genes.

However, since the above-mentioned methods are based on the detection of transcriptional activation, they have several drawbacks such as the generation of false positive responses, since many proteins containing transactivation as well as DNA binding domains can activate the system without a respective interaction. Furthermore, since transcription occurs in the nucleus, the above-mentioned methods are neither appropriate for investigating proteins which are toxic to cells when expressed in the nucleus nor proteins mainly acting in the cytoplasm, because of which those proteins obviously cannot be analyzed.

Hence, there is still a need for assays detecting interactions between proteins and peptides under physiological conditions in living cells.

### Summary of the invention

The present invention overcomes the drawbacks of the prior art by providing the embodiments characterized in the claims and described further below. In particular, the present invention provides a method which is easy to perform and does not only allow for investigation of cytosolic proteins, transcription factors and small peptides but also provides a method which by its easy way to handle is suitable for being used in high throughput screening. The simplicity/convenience of the method of the present invention due to for example the use of cell growth as altered phenotype upon protein-peptide interaction, i.e. as signal indicating whether or not the intended interaction of interest has occurred allows an easy detection of effect/result and renders it advantageous over the prior art.

In general, the present invention concerns methods for detecting interactions among proteins and peptides. Accordingly, in one aspect, the present invention relates to a method of determining whether a first protein is capable of physically interacting with a second protein, i.e. peptide within a cell featuring the use of a fusion of a target protein and an effector molecule, and a peptide which is conformationally stabilized by linkage to a carrier protein and directed through fusion with an additional domain to a particular cell compartment. The method and assay of the present invention combines the reliability of biochemical in vitro or simple two hybrid assays for protein-protein/peptide interactions and the validity of complex in vivo investigations aiming at elucidating the interaction of a given protein with its binding ligand.

Since usually the target protein will be predetermined while the interacting peptide(s) remain to be identified the target protein may also be considered as "bait" and the interacting peptide(s) as "prey". However, the person skilled in the art will immediately recognize that the method of the present invention can also be performed vice versa. Those skilled in the art will also recognize that the bait and prey protein/peptides may be derived from any appropriate eukaryotic or prokaryotic source, including yeast, mammalian cell, and prokaryotic cell genomes or cDNAs as well as artificial sequences. The present invention further provides a drug screening assay useful for identifying a drug that can alter a particular protein-protein/peptide interaction or modulate the activity of a target protein. In addition, present the invention provides a kit useful for performing the screening assay.

### Brief description of the drawings

The figures illustrate the invention and show:

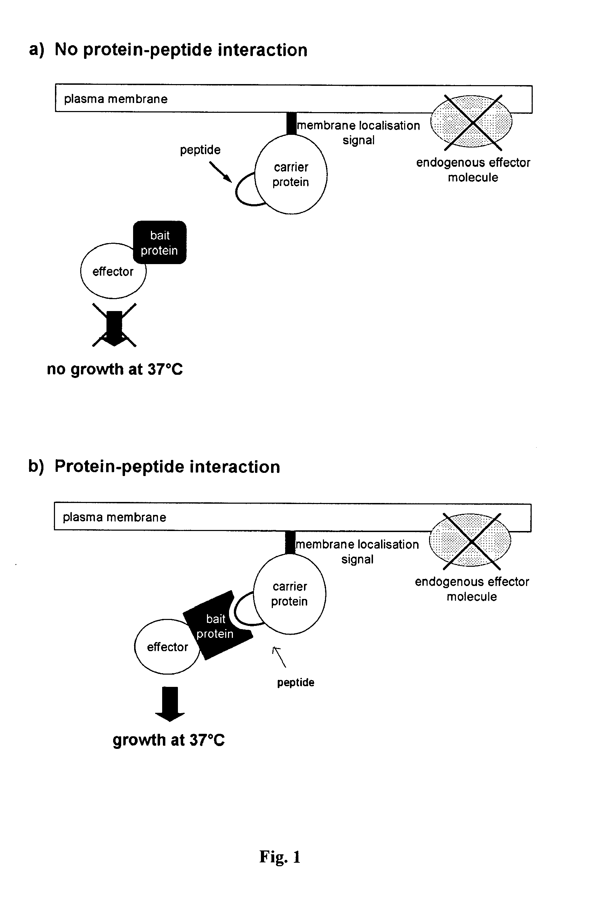

- **Fig. 1**:: illustrates schematically the analysis of a protein-peptide interaction in cells according to the method of present invention.

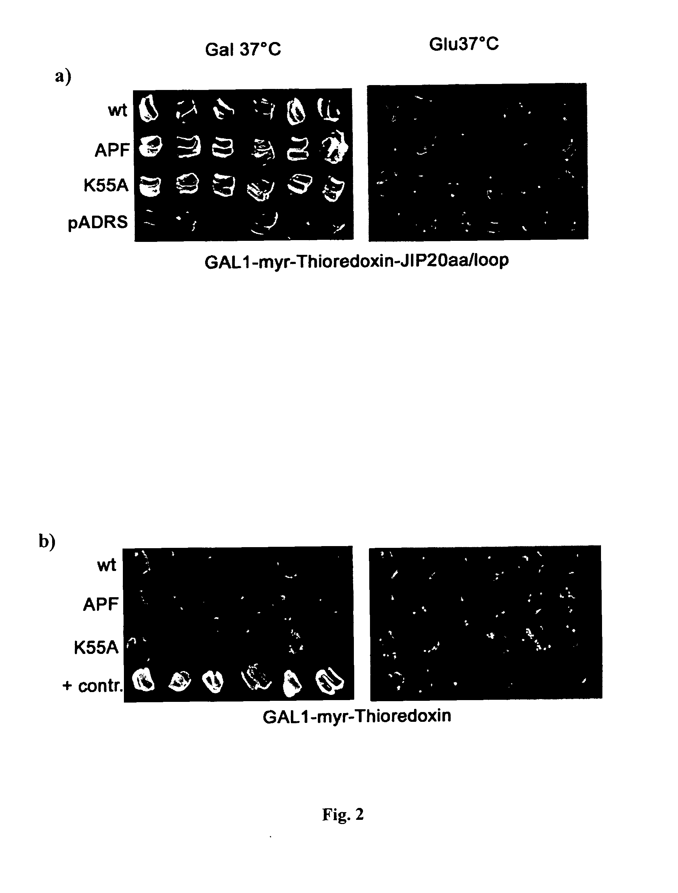

- **Fig. 2**:: generally illustrates growth of yeast cells in dependency of the simultaneous expression of two fusion proteins (fusion protein 1, the "bait", comprising the Ras-protein and JNK and fusion protein 2, the "prey" comprising a membrane localization domain, the carrier protein thioredoxin and the peptide JIP20, intended to intract with the JNK upon binding of "bait" and "prey". In particular, Fig. 2 the interaction of the "bait" which is expressed in context of fusion protein 1 comprising the protein of interest and a respective effector and the "prey", i.e. the peptide of fusion protein 2, which is localized to cell-membrane. Ras protein as the exemplified effector used in the Examples of the present invention is recruited to the compartment (here: plasma membrane) where the "prey" which is intended to interact with the "bait" is localized due to its membrane localization domain. Upon recruitment of Ras (bound to "bait") to the membrane, Ras-pathway is activated which results in a detectable phenotyp such as cell growth of yeast cells at a restrictive temperature of 37°C.
(a) illustrates the growth of yeast Cdc25-2 yeast cells at a restrictive temperature (37°C) as a function of the interaction of the protein c-jun N-terminal kinase (JNK) and the peptide JIP20, in the course of which the Ras protein fused to the protein (JNK) is recruited to the membrane and as a consequence activating the Ras signalling pathway, allowing the yeast cells to grow even at the restrictive temperature. Cell growth is shown in dependency of the simultaneous expression of both fusion proteins, the expression of which is induced by galactose, since it is regulated by a GAL1-promoter. As expected, cell growth at the restrictive temperature is only detected upon galactose addition (left part) whereas addition of glucose did not show any cell growth (right part). Thus, growth only occurs after Ras is recruited to the membrane.
   Hence, after induction of gene expression by addition of galactose, both fusion proteins (protein 1, comprising Ras and protein 2, comprising the location domain) are expressed, allowing for an interaction of JNK with JIP20 and as a result the recruitment of Ras to the membrane where it exerts its effect in activating the Ras signalling pathway as a result of which the phenotype of cells is altered in that they can grow even at restrictive temperature of 37°C. The abbreviations wt, APF, K55A represent different JNK variants and pADRS represents a control, i.e. empty vector; for details see Example 1.
(b) illustrates a control experiment were cells were with JNK1 constructs identical to (a), but with a different thioredoxin-JIP20 construct (empty vector), i.e. a construct, entirely lacking JIP20. As demonstrated, cell growth is dependent on the expression of JIP20 but not on the expression of thioredoxin alone, since no cell growth is detectable, again proving that the expression of JIP20 and its interaction with JNK, respectively as well as the subsequent recruitment of Ras to the membrane accompanying this interaction is important. Since no interaction of JNK1 with JIP20 was possible due to lack of JIP20, no cell growth is detectable. A general failure of this approach was excluded by applying a "positive control", i.e. proteins, the expression of which is also induced by galactose and the interaction of which is known to lead to the respective cell growth, those proteins in the present case being cyclin dependent kinase (CDK4) and cyclin dependent kinase inhibitor (p16). Again, as expected, addition of glucose did not induce the thioredoxin expression and did not lead to the growth signal.

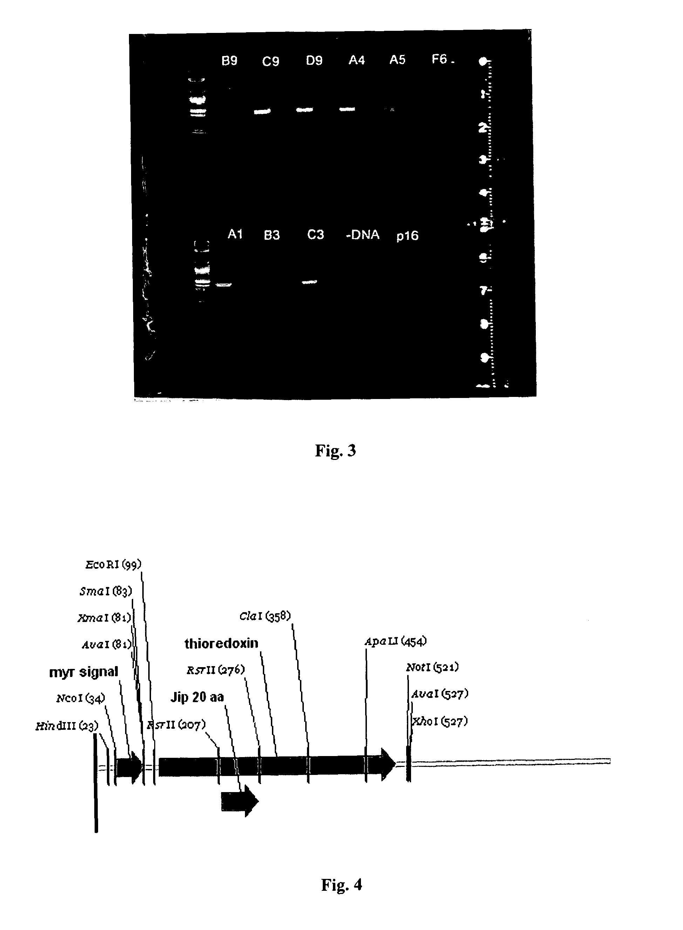

- **Fig. 3**:: shows a photo of an agarose gel. DNA fragments, i.e. PCR products amplified with primers specific for thioredoxin and JIP 15, respectively, are shown representing yeast clones being positive for the existence of a thioredoxin fragment as well as for the existence of a JIP 15 fragment; for detail see also Example 2. As demonstrated, clones B9, C9, D9, A4, A5, A1 and C3 were positive for the existence of a thioredoxin fragment as well as for the existence of a JIP 15 fragment. However, although a positive PCR result was not obtained for clones B3 and F6, they were also defined as positive according to the results in growth analysis. Those clones could express interacting proteins from the Hela library.
- **Fig. 4**:: schematically represents a linearized plasmid based on pYES (Invitrogen). The sites of inserted myristoylation signal, JIP20 and thioredoxin, respectively, as well as restriction sites are indicated.

### Definitions:

"conformationally stabilized" as the term is used herein, generally refers to a peptide or protein, having reduced flexibility due to its amino and carboxyl terminus, respectively, fixed in space, wherein the conformationally stabilized peptide or protein is preferably presented in a structurally rigid manner. Conformational stabilization can be facilitated by embedding the protein or peptide of interest within a conformation-stabilizing molecule such as a conformation-stabilizing protein, for example a carrier-protein.

"candidate peptide" and "candidate protein", respectively, as the term is used herein, generally describes a peptide and a protein, respectively, being a candidate for an interaction with a partner of interest.

"agonist" and "antagonist", as the term is used herein, generally describes an interacting molecule having the ability to increase (agonist) or decrease (antagonist) production of the respective signal.

"randomly generated", as the expression is used herein, generally relates to sequences being not predetermined.

"intentionally designed", as the term is used herein, generally refers to sequences having a certain DNA or protein sequence or motif determined prior to their synthesis.

### Detailed description of the invention

The present invention provides a method for detecting protein-peptide interactions *in vivo* and isolating the respective interaction partner(s). More specifically, the method of the present invention is based on detecting molecular interaction of a target protein "bait" which is linked to an effector molecule thereby generating a hybrid molecule, and a peptide "prey" or a population of potential interacting peptides which are conformationally stabilized by linkage to a carrier protein which contains a cell compartment localization domain. In particular, this interaction is detected by a signal that is induced by the respective effector molecule which by binding of "bait" and "prey" is recruited to a specific cell compartment, wherein the recruitment is due to locally restricted presentation of the "prey" at the respective cell compartment where the effector then exerts its effect.

As demonstrated in the examples, the present invention provides an "easy to perform"-method, capable of specifically detecting protein-peptide interactions by inducing an effector-mediated signal, such as cell growth; see also Fig. 2. Furthermore, Fig. 2 illustrates the specificity of the protein-peptide interaction as well as the simplicity of its detection by monitoring cell growth. As cell growth is detectable upon addition of galactose the signal is specific and easy to detect and interpreted. It is a simple "yes or no"- answer not requiring any gradual analyses. Hence, the present invention significantly contributes to the investigation of protein and peptide-ligand interaction which hitherto was difficult if at all to be accomplished.

Thus, in one aspect the present invention relates to a method of identifying a protein-peptide interaction or isolating a partner of a protein-peptide interaction, comprising:
(a) providing within a host cell
   (i) a hybrid molecule, comprising a target protein and an effector;
   (ii) a peptide or a population of peptides, wherein the peptide is linked to a carrier protein, said carrier protein being linked to a cell compartment localization domain; and
(b) detection of a signal identifying the protein-peptide interaction of the hybrid molecule and the peptide; and optionally
(c) isolating a partner of the protein-peptide interaction based on its ability to alter the signal when present, preferably wherein the hybrid molecule is a fusion protein.

Effectors and effector molecules, respectively, are well known to mediate effects, which in their absence do not or only less occur. Mediating comprise a plurality of processes such as providing efficiency and specificity in cell signalling itself as well as in the coordination of signalling processes and in this context, especially compartmentalized signalling, i.e. the transfer of a signal from one cell compartment to another. Although there is a broad range of effector molecules having localized activity, such as scaffold, -anchor- and adaptor proteins, effector molecules which are especially involved in compartmentalized signalling comprise for instance G-proteins, protein kinases, protein phosphatases and Ras-proteins, just to name a few, which mediate for example activation of the phosphatidylinositol 3-kinase pathway, the compartmentalized cAMP signalling and the Ras/MAPK signal transduction pathway.

As known to the person skilled in the art, detecting such a mediated effect requires the absence of the same, i.e. of said effect, if the effector is not present. Thus, in a further preferred embodiment, the host cell used in the method of the present invention is lacking an active endogenous effector.

A further advantage of the method of the present invention is that numerous effector molecules are applicable. One component of the method and assay of the present invention, i.e. the recruitment of an effector protein, which is not a transcription factor, to a particular cell compartment, where the effector protein can activate a reporter molecule is disclosed US patent no. 5,776,689. The protein recruitment system is exemplified therein using yeast cell based assay, in which a protein-protein interaction results in the recruitment of a guanine nucleotide exchange factor (GEF) to the plasma membrane, where the GEF activates a Ras reporter molecule, resulting in the survival of cells that otherwise would not survive under the particular cell culture conditions. Accordingly, while the method and assay of the present invention does consist of the protein recruitment system described in US patent no. 5,776,689 per se, the disclosure content of this application as well as that of the publications cited therein is incorporated herein by reference for the purpose of providing information on one or elements of the present invention, in particular target proteins such as Ras and effector molecules such as guanine nucleotide exchange factor that is known to the person skilled in the art including nucleotide and amino acid sequences of the appropriate proteins. In this context, unless otherwise stated the term "effector molecule" and "effector protein", respectively, is used as defined and explained in this US patent. The same applies to the "active endogenous effector protein" and the lack of it, respectively. In addition, as used herein, the term "cell compartment" and "cell compartment localization domain" equate to the corresponding terms in US patent no. 5,776,689 which also contains ample examples of useful domains that can be applied in accordance with the present invention as well; see for example the amino acid sequences of SEQ ID NOs. 1 to 3 therein. Accordingly, also for the purpose of providing information on cell compartment localization domains the disclosure content of US patent no. 5,776,689 and the publications cited therein is incorporated herein by reference. Likewise, appropriate cells and cell lines, e.g. *S*. *cerevisiae* cdc25-2 cells and NIH 3T3 cells are described. In addition, this US patent contains useful information on the identification and elimination of "false positives", which for this purpose is also incorporated herein by reference.

The "Sos Recruitment System" (SRS) and particularly the Ras Recruiting System (RRS), the latter making use of cells which are not able to express functional Ras due to the fact that the GTP/GDP exchange factor of Ras is mutated such that it cannot be localized at the membrane, e.g. lacking the farnesylation box or having a mutation therein are described in international application WO00/05410 and references cited therein, the disclosure content of which by reference for the purpose of providing information on RRS and components thereof that is known to the person skilled in the art including nucleotide and amino acid sequences of the appropriate proteins, cloning and expression vectors, cells such as yeast cdc25 mutant strains, culture conditions, etc, for performing the protein-protein interaction assay described therein, and which can be used and adapted in accordance with the present as well.

Hence, guanine nucleotide exchange factor (GEF), preferably the Sos-protein, preferably the human Sos-protein (h-Sos) can be used as effector molecule. In another preferred embodiment, the effector used in accordance with the method of the present invention comprises the Ras-protein, preferably the human Ras-protein (h-Ras). Most preferably, a mutated Ras-protein which cannot localize to the cell membrane and does not require an exchange factor is used as the effector molecule, e.g., a Ras-protein lacking a farnesylation box, the latter of which is also exemplified in the Examples conducted in accordance with the present invention. Since the Ras-protein is a GTP-bound Ras-protein (Q61L) without membrane localization domain (ΔF), only in case of a molecular interaction of the target protein with the peptide and as consequence recruitment of Ras to the membrane, a detectable Ras-mediated signal transduction at the plasma membrane is detectable. Therefore, in the preferred embodiments the detectable signal will be due to the activation of the Ras-protein.

As it is well known in the art and as already mentioned above, for the detection of an effect, which is mediated by an effector, the absence of said effect is mandatory in the absence of the effector. Hence, for detecting activation of for example the Ras signalling pathway, it is obvious that the pathway should not be activated without the effector. Therefore, organisms, cells, cell lines or cultures, known to those skilled in the art have to be prepared in that they lack the possibility to self-activate the Ras signalling pathway and therefore to be dependent on an effector such as Ras protein, which according to the present invention is only recruited to its site of action, i.e. the membrane, upon the aforementioned interaction of a target protein being fused to a Ras protein lacking the ability to localize itself to the membrane, and a peptide which is linked to a protein having a membrane localization domain and thereby being localized at the membrane.

One major advantage of the method of the present invention is that the activation of the Ras signalling pathway upon the aforementioned interaction can be easily detected by for example an altered phenotype of a host cell, such as growth of cells, which are under restrictive conditions defective in producing a detectable Ras signal without the above described interaction. In this context, one major advantage of the method according to the present invention is that the use of Ras-proteins as effector molecules, having no DNA binding or transcriptional activity, allows for the investigation of transcription factors as target proteins, since in comparison to DNA binding domains as effectors and transcriptional activation as readout, those hybrid molecules are not auto active without interaction.

A further major advantage of the method of the present invention is that it can be applied to almost any type of cell, such as mammalian, avian, insect, yeast and *E. coli* cells. However, in one preferred embodiment, the host cell used in the method according to the present invention comprises an eukaryotic cell, preferably a yeast cell; see also supra. Thus, while yeast cells are preferred a plurality of other eukaryotic cells can be used, in particular mammalian cells such as CHO, HEK, COS7, 3T3, or 293 cells.

As it is known, within a cell Ras proteins are present in their inactive form, i.e. bound to GDP (Ras-GDP) showing no signalling activity. By contrast, in its active form, i.e. bound to GTP (Ras-GTP) those proteins mediate signalling processes from the plasma membrane to the nucleus, wherein the activation of Ras is controlled by guanosine exchange factors (GEFs), which convert inactive Ras-GDP to active Ras-GTP. As discussed above, if activation of the Ras signalling pathway is the signal to be detected it is suitable to use cells being devoid of functional Ras and therefore having a phenotype which is altered depending on the presence and absence of Ras. Thus, the cells used in the method of the present invention are defective in providing a functional Ras.

As mentioned above, to investigate the effect of protein-peptide interaction, i.e. the recruitment of Ras to the membrane, it is necessary to use cells the phenotype of which changes upon Ras-recruitment to the membrane. Therefore, in the examples of the present invention yeast cells were used being mutated in cdc25 (cdc25-2), Cdc25 being a GEF that when localized at the plasma membrane, leads to the activation of Ras; see Petitjean et al., Genetics 124 (1990), 797-806. The temperature sensitive Cdc25-2 mutation, however, cannot express a functional Ras effector, i.e. cannot activate Ras at the restrictive temperature of 37° leading to cells having a phenotype of a growth defect at the respective temperature, a phenotype which is also found in other organisms like *S*. *pombe,* Drosophila and different mammalian cells. The person skilled in the art will easily know how to "generate" a defective effector, for example either by mutating the effector itself of by mutating proteins that influence its activity as well as also changes in the expression level as well as external conditions can influence effector activities. However, this defect can be overcome by Ras-activation upon the protein-peptide interaction, described in the examples of the present invention.

Thus, in a particularly preferred embodiment of the method of the present invention, the yeast cells are *Saccharomyces cerevisiae* cdc25-2 cells. Especially in this embodiment, the detectable signal is preferably cell growth at the non-permissive temperature of the host cell, for example at 33 - 37°C. However, besides the phenotype of cell growth, the skilled artisan will easily understand that there are several phenotypes and effects, respectively which depending on the experimental setup can be altered, i.e. represent a measurable change in response to an interaction between a "bait" molecule and a "prey" molecule, such as accumulation of substances, activation of genes as well as any changes in fluorescence activity.

As mentioned, the present invention provides a method for detecting peptide-protein interaction as well as identifying and isolating novel candidate peptides for interaction with a respective target protein. Although the method of the present invention is useful for investigating a wide variety of different therapeutic target proteins, in a preferred embodiment the target protein used in the method of the present invention is selected from the group consisting of kinases, viral proteins, nuclear receptors, transcription factors, membrane proteins, phosphotases, ubiquitinating enzymes, and preferably comprises a kinase, more preferably a c-Jun N-terminal kinase (JNK), most preferably a mutated JNK, such as JNK APS, JNK K55A, or JNK wt.

As mentioned above, the target protein exemplified in the Examples of the present invention, is the c-Jun N-terminal kinase (JNK) which is a member of the family of serine and threonine mitogen-activated protein kinases and is involved in signal transduction of numerous physiological processes, including tissue differentiation and pathogenesis. To date, three genes encoding for JNKs have been identified and ten isoforms resulting from alternative splicing of these genes have been described. JNKs have been implicated in several important diseases including cancer, diabetes, and neurodegenerative diseases.

In the context of the experiments conducted in accordance with the present invention, the interaction of JNKwt (JNK1α2), as well as that of different mutants of JNK with the peptide JIP20 having the sequence GPGDTYRPKRPTTLNLFPQVPRSG (SEQ ID NO: 1) could be detected by the method of the present invention; see Example 1

The person skilled in the art will easily recognize, that the application of the present method is not restricted to this example and that also other protein-peptide interactions are well detectable and may prove the applicability of the method of the present invention, such as for example the interaction of the human androgen receptor as target protein with the peptide 622, the interaction of which is known and described in for example Chang et al., Mol. Endocrinol. 19 (2005), 2478-2490. The human androgen receptor is a member of the family of nuclear hormone receptors, i.e. a family of ligand dependent transcription factors which are key regulatory proteins in diverse physiological processes including embryogenesis, sexual development, energy homeostasis, and fat metabolism.

Statistical evaluations of amino acids involved in protein-peptide interactions strongly suggest that proteins recognize and interact with peptides preferably through a restricted set of specialized interface amino acid residues, such as Pro, Ile, Tyr, Trp, Asp and Arg (Sillerud and Larson, Current protein and peptide science 6 (2005), 151-169), since these amino acids represent residues from each of the three classes of amino acids, i.e. hydrophobic, aromatic and charged, having one anionic and one cationic residue at neutral pH. In this context, the use of a carrier protein turned out be a further major advantage of the method of the present invention ensuring a functionally stabilized presentation of the peptide and preventing disturbing interactions of the peptide with the respective cellular compartment, e.g. the plasma membrane, such as the interaction of polar amino acids with hydrophilic head groups of the phospholipid membrane or insertion into it. Additionally, the carrier protein is able to provide protection from proteolytic degradation.

For the purposes of the present invention, especially small proteins of known structure are useful as carrier proteins. As exemplified in the Examples of the present invention, in a preferred embodiment, the carrier protein used in accordance with the method of the present invention is thioredoxin or a thioredoxin-like molecule, although other molecules such as green fluorescent protein, Z-domain of protein A, tendamistat, Kunitz domain, fibronectin type III domain, lipocalin, basic pancreatic trypsin inhibitor and conotoxine can also serve as carrier; see Souriau et al., Biochem 44 (2005), 7143-7155.

As demonstrated in the examples thioredoxin and thioredoxin-like conformation-constraining proteins are the most preferred carrier proteins to be used in the method of the present invention. Unless indicated otherwise, the term "conformation-constraining protein" is used in accordance with the definition provided in international application WO96/02561 meaning any peptide or polypeptide which is capable of reducing the flexibility of another protein's amino and/or carboxy termini. Preferably, such proteins provide a rigid scaffold or platform for the protein of interest. Examples of conformation-constraining proteins include thioredoxin and other thioredoxin-like proteins as disclosed for example in mentioned international application WO96/02561. Accordingly, while the method and assay of the present invention does consist of the protein-protein interaction system described in international application WO96/02561 per se, the disclosure content of this application as well as that of the publications cited therein is incorporated herein by reference for the purpose of providing information on thioredoxin-like proteins that is known to the person skilled in the art including nucleotide and amino acid sequences of the appropriate proteins. Furthermore, the utility of thioredoxin or other thioredoxin-like proteins is described in US patent no 5,270,181 and by LaVallie et al., Bio/Technology 11 (1993), 187-193. These two references are hereby incorporated by reference. In preferred embodiments the intracellular peptide is embedded within the conformation-constraining protein, preferably thioredoxin; the intracellular peptide is conformationally-constrained by disulfide bonds between cysteine residues in the amino-terminus and in the carboxy-terminus of said second protein; the population of eukaryotic cells are yeast cells; the recombinant DNA molecule further encodes a gene activating moiety covalently bonded to said intracellular peptide; and/or the intracellular peptide physically interacts with a second recombinant protein inside said eukaryotic cells.

According to the present invention, for detecting protein-peptide interactions the peptides can either be directly linked to the C-terminus or N-terminus of the carrier-protein, or alternatively be inserted in the carrier protein through disulfide bonding of suitably located cysteine residues and for example be presented as inserts in the active-loop of thioredoxin.

For the sake of information it is noted that in one embodiment, the method and assay of the present invention does not comprise or consist of the split-ubiquitin system as specifically disclosed and claimed in international applications WO02/27020 and WO037083136. However, the description of these applications may contain useful information on molecular biological tools and materials such as vectors, cells, nucleic acid library construction, compound libraries and collections, etc., which may employed in accordance with the teaching of the present invention. Accordingly, the disclosure content of international applications WO02/27020 and WO03/083136 as well as that of the publications cited therein is incorporated herein by reference for the purpose of providing information on the appropriate proteins, cloning and expression vectors, cells, culture conditions, etc, for performing and analyzing the result of protein-protein interaction assays. Furthermore, the disclosure content of international application WO03/083136 is particularly incorporated herein by reference for the purpose of information on the identification/design of scFVs or antibodies that are capable of interfering with protein-protein interactions and the identification of compounds that bind to and activate G protein-coupled receptors (GPCRs) or that selectively bind to and activate one GPCR but not another GPCR that is co-expressed within the same cell.

Furthermore, since the present invention does not necessitate the use of a transcription factor as the effector protein, preferred embodiments of the present invention are distinguishable from the two hybrid assay; see Fields and Song, Nature 340 (1989), 245-246; US patent no. 5,283,173, each of which is incorporated herein by reference and variations thereof; see, for example, Fearon et al., Proc. Natl. Acad. Sci., USA 89 (1992), 7958-7962, and Osborne et al., Biotechnology 13 (1995), 1474-1478, each of which is incorporated herein by reference. Furthermore, the protein-peptide interaction assay of the present invention involves the translocation of an effector protein to the cell compartment containing a reporter molecule, thus further distinguishing the invention from the two hybrid assay.

As mentioned hereinbefore, another component of the method of the present invention is directing the putative interacting peptide(s) to a particular cell compartment, for example through the use of a corresponding cell compartment localization domain linked to peptide via the carrier protein, preferably within a fusion protein. Cell compartment localization domains in general mediate localization of the respective molecule, such as peptide or protein to a predetermined compartment. However, this can be any compartment within a cell, to which a respective protein can be localized. There are various compartments known to the person skilled in the art such as the plasma membrane, the nucleus, mitochondrial membrane, the endoplasmatic reticulum (ER), the Golgi apparatus, as well as cell compartments of plant cells like the vacuole, and the chloroplast. To render an intended molecule localized at the respective compartment, the molecule of interest is usually provided with a respective domain, enabling the aforementioned localization, i.e. to for example attach to or integrate in a certain compartment or parts thereof.

Various cell compartment localization domains are known to the skilled person such as membrane localization signals, nuclear localization signals, and ER- or a mitochondrial localization sequences, just to name a few; see for example Nakai and Kanehisa, Genomics 14 (1992), 897-911. The cell compartment localization domain which according to the method of the present invention is linked to the carrier protein, either N- or C-terminally, determines the accessibility of the peptide or population of peptides presented. As exemplified within the Examples of the present invention, the cell compartment localization domain comprises a polypeptide sequence that directs translocation of the carrier-peptide hybrid molecule to a particular cell compartment. In a preferred embodiment, the cell compartment localization domain used in accordance with the method of the present invention is a membrane localization domain, preferably a plasma membrane localization domain and most preferably a myristoylation signal.

As already mentioned above, the investigation of protein-peptide interactions comprising a short peptide as candidate, i.e. as a potential interacting partner, is difficult due to sterical hindrance of the interaction or even insertion of the peptide into the membrane. Therefore, the present invention is especially suited to also investigate those interactions, since the binding of a short peptide or its embedding in a carrier protein not only prevents from the aforementioned drawback by providing a "spacer", but also conformationally stabilizes the peptide.

Although peptides with similar amino acid sequences have already been shown to interact with JNK using alternative experimental methods (Barr et al., J Biol. Chem. 277(13) (2002), 10987-10997, Barr et al., J Biol. Chem. 279 (41) (2004), 42178-42189), the detection of interaction of JNKwt as well as selected JNK-mutants and the peptide JIP20 GPGDTYRPKRPTTLNLFPQVPRSG (SEQ ID NO: 1) demonstrates that the method of the present invention allows for detection of specific binding to even small peptides, especially regarding the fact that JIP 20 comprises 20 amino acids of the sequence of the JNK interacting protein 1 (plus 4 amino acids of linker), containing a number of charged (Arg, Lys, Asn, Gln) and hydrophobic (Leu, Val, Phe) amino acid residues which in another method being devoid of the advantages of the present invention. i.e. having no carrier molecule to which the peptide is linked could result in unspecific interactions with the plasma membrane and other cell compartments. As demonstrated in Example 1, unintended and unspecific binding is prevented by the use of a carrier molecule in the method of the present invention. In a particular preferred embodiment the peptide used in accordance with the method of the present invention is imbedded within the carrier protein.

The person skilled in the art will easily understand that a broad variety of peptides are suitable to be used in the present invention. Therefore, in one embodiment the peptide used in accordance with the method of the present invention comprises a randomly generated or (intentionally) designed sequence. In another embodiment, a peptide is used comprising 10 to 30 amino acids in length, preferably 15 to 25 amino acids, and more preferably 20 to 25 amino acids. In one particular embodiment the peptide comprises or consists of the amino acid sequence GPGDTYRPKRPTTLNLFPQVPRSG (SEQ ID NO: 1). However, the skilled artisan will recognize that also library encoded peptides can be used in the method of the present invention

Random or designed peptide-encoding libraries may be used in accordance with the present invention for selecting and screening, respectively, as well as for identifying candidates of novel protein-interacting peptides. Respective libraries or known to the skilled artisan; see also the prior art cited supra. As exemplified in Example 2, the method of the present invention can be used for identifying interacting peptides within a population of DNA encoded proteins or peptides, wherein the population can comprise randomly generated as well as designed peptides. Thus, in a further preferred embodiment, the present invention relates to a method of detecting and/or isolating an interacting peptide in a population of peptides comprising:
(a) providing a hybrid molecule, comprising a target protein and an effector within a host cell;
(b) introducing a test peptide or a population of test peptides into the host cell, wherein the peptide is linked to a carrier protein, said carrier protein being linked to a cell compartment localisation domain;
(c) detection of a signal identifying the protein-peptide interaction of the hybrid molecule and the peptide; and optionally
(d) isolating a peptide, identified as a binding partner based on its ability to alter the signal when present.

For details of appropriate host cells, target protein, effectors, peptides, carrier proteins, compartment localisation domains as well as a detectable signal see supra and the Examples.

Beside peptide (JIP20), which is exemplified in the Examples, the present invention also employs peptides and population of peptides being encoded by a nucleic acid library, for example a cDNA or EST library. It is known that peptide-encoding DNA-libraries can be designed based on information about binding preferences of the target protein. Therefore, in order to find novel candidates of interacting peptides or to identify peptides with an improved affinity to the target protein, keeping certain amino acids of the library peptides at constant positions is suggestive, since some amino acids could represent an important part of a binding motif, which may already be known. Different examples for the use of biased peptide libraries have been described in the literature; see for example Pero et al., Int. J. Cancer: 111 (2004), 951-960; Rajagopal et al., Bioorg. Med. Chem. Letters 14 (2004), 1389-1393; O'Boyle et al., Virology 236 (1997), 338-347; Linn et al., J Biol. Chem. 378 (1997), 531.

Furthermore, populations of peptides can be enriched for peptides with for example a desired property such as hydrophobicity, net charge, polarity or side chain size. Methods for quantitative codon optimization of respective DNA libraries are incorporated herein by reference; see for example Larsson et al., Nucleic Acids Res. 30: e133 (2002); Park et al., Computers Chem. Engineering 29 (2005), 407-421.

Moreover, DNA-libraries deriving from the genome of specified organisms can also be used to encode peptide populations. Methods for generating peptide libraries using genomic DNAs from different species have been described in the literature; see Huang and Gao, Mol Biotechnology 30 (2005), 135-142. Furthermore, methods for constructing expression libraries deriving from micro-organisms and eukaryotes containing compact genomes are described in for example international application WO2004/074479.

As already indicated in the preceding sections, the method of the present invention can also be used in the reverse, i.e. identifying a desired target protein. In this embodiment, the peptide(s) will preferably be pre-determined. For example, peptide-ligands may be used, which are known to interact with a family of GPCRs but for which a further member is postulated and remained to be identified. With the help of the method of the present invention it shall be possible to identify and isolate such further and eventually more specific GPCRs. Thus, in yet another embodiment the present invention relates to a method for isolating a protein comprising:
(a) providing a host cell comprising a test protein or a population of test proteins and an effector, or introducing the effector;
(b) introducing the linkage of the effector to the test protein or population of test proteins;
(c) providing or introducing a peptide or population of peptides within/into the host cell, wherein the peptide is linked to a carrier protein, said carrier protein being linked to a cell compartment localisation domain;
(d) detection of a signal identifying the protein-peptide interaction; and
(e) isolating the protein identified as a binding partner based on its ability to alter the signal when present

The cell based systems described in the above examples may also be used to identify agonists or antagonists, simply by adding to a known pair of interacting proteins (in the above described system) a candidate agonist or antagonist interactor and assaying for an increase or decrease (respectively) in reporter gene expression, as compared to a control reaction lacking the candidate compound or protein. Particular examples of interacting proteins for which antagonists or agonists may be identified include, but are not limited to, the IL-6 receptor-ligand pair, TGF-ss receptor-ligand pair, IL-1 receptor-ligand pair and other receptor-ligand interactions, protein kinase-substrate pairs, interacting pairs of transcription factors, interacting components of signal transduction pathways (for example, cytoplasmic domains of certain receptors and G-proteins), pairs of interacting proteins involved in cell cycle regulation, and neurotransmitter pairs.

Hence, the present invention also relates to a method for identifying and optionally isolating an antagonist or agonist molecule comprising:
(a) providing or introducing within/into the host cell
   (i) interacting proteins within/into a host cell partially/some of which comprising hybrid molecules, which comprise a target protein and an effector;
   (ii) a candidate agonist or antagonist molecule;
   (iii) a peptide or a population of peptides, wherein the peptide is linked to a carrier protein, said carrier protein being linked to a cell compartment localization domain
(b) detecting
   (i) a signal identifying the protein-peptide interaction of the hybrid molecule and the peptide; and optionally
   (ii) the alteration of the signal based on the ability of an agonist molecule to increase production of the signal or the ability of the antagonist to decrease production of the signal; and optionally
(c) isolating the agonist or antagonist molecule.

To facilitate large scale screening, candidate target proteins or candidate agonist or antagonists may be initially tested in pools, for example, of ten or twenty candidate compounds or protein. From pools demonstrating a positive result, the particular interacting protein or agonist or antagonist is then identified by individually assaying the components of the pool. Such systems are amenable to robotic automation or to the production of kits. Kits including the component of any of the interaction assay described herein are also included in the invention. The components (e.g., the various fusion proteins or DNA therefor) of any of the in vivo or in vitro systems of the invention may be provided sequentially or simultaneously depending on the desired experimental design.
The assay system of the present invention can also be used to test affinity reagents for protein purification. Peptides or protein domains can be identified that interact with the known membrane protein of interest and these may then be used in a purification protocol for the known protein.

In a further aspect, the present invention also relates to the peptides and proteins obtainable by the methods of the present invention as well as to the agonists and antagonists so identified. In one particular embodiment the peptide comprises or consists of the amino acid sequence depicted in SEQ ID NO: 1. Naturally, the present invention also relates to the nucleic acid molecules encoding the aforementioned peptides, proteins, agonists and antagonists. Additionally, agonists or antagonists can be expressed from a separate expression system to modulate the present system of invention. These agonists or antagonists can be encoded by nucleic acid molecules or supplied as compounds.

The present invention also provides a kit for performing the method and assay of the present invention. Such a kit comprises the essential components such as for the target protein and peptide or collection of peptides, preferably in the form of a corresponding first and second expressible nucleic acid molecule, respectively, see also supra and the examples. In general, the expressible nucleic acid molecules are present in an expression vector suitable for the particular cells in which the interaction assay is performed. Appropriate expression vectors can be, for example, yeast expression vectors or mammalian cell expression vectors, depending on the cells in which the protein recruitment system is to be practiced. Each of the first and second expressible nucleic acid molecules generally contains a cloning site such as a multiple cloning site, which permits a convenient means to insert a nucleic acid molecule encoding a target protein or a peptide(s), respectively. In particular, the cloning site permits insertion of a nucleic acid such that the encoded protein is in frame with the effector protein or with the carrier protein and cell compartment localization signal, respectively, which can constitute the N-terminus or the C-terminus of an encoded fusion protein. In addition, the expressible nucleic acids can contain appropriate transcription or translation start or stop signals or the like.

If desired, such a kit can contain reagents, for example, that result in optimal transfection efficiency of the nucleic acids for the particular host cell type. In addition, appropriate host cells can be included in a kit, although such cells generally are available or can be selected for a particular embodiment of the interaction assay system. Preferably, the kit of the present invention contains reagents such as those described hereinbefore useful for conducting any of the above-described methods of the present invention, comprising medium or media components, reference samples, micro arrays, culture vessels, cell suspending media, vectors, proteins, peptides, or the like. Such a kit would typically comprise a compartmentalized carrier suitable to hold in close confinement at least one container and the compounds of the kit may be sterile, where appropriate. The kit may further include a transfer means, such as pipets, for transferring any fluent component. A further possibility includes the direct transfer of the kit components to solid media, like agar containing media by mechanical transfer devices, like streaking or gridding tools.

In one particular embodiment, the kit of the present invention comprises:
(a) a cell or a culture comprising cells as defined above;
(b) a first nucleic acid vector for inserting a DNA sequence encoding a fusion protein which comprises a target protein and an effector as described supra;
(c) a second nucleic acid vector, for inserting a DNA sequence encoding a peptide and a carrier protein as defined hereinbefore;
(d) reagents and devices for transfecting the cells with the first and the second nucleic acid, and
(e) a monitoring arrangement for monitoring the signal due to protein-peptide interaction as defined supra.

The kit's carrier could further comprise reagents useful for performing said methods and may also contain means for detection. Instructions can be provided to detail the use of the components of the kit, such as written instructions, video presentations, or instructions in a format that can be opened on a computer (e.g. a diskette or CD-ROM disk). These instructions indicate, for example, how to use the method to screen for candidate peptides.

The present invention further relates to the use of the aforementioned individual components such as target proteins, carrier proteins, peptides and their encoding nucleic acid sequences, host cells, etc. in any one of the methods of the present invention. Preferably, the DNA encoding the peptide used in the methods of the present invention is randomly generated or (intentionally) designed.

It will be apparent that the methods of the present invention, the peptides obtained thereby, as well as the uses as substantially described herein or illustrated in the description and the examples, are also subject of the present invention and claimed herewith. In this respect, it is also understood that the embodiments as described in any one of the examples, can be independently used and combined with any one of the embodiments described hereinbefore and claimed in the appended claims set.

These and other embodiments are disclosed and encompassed by the description and examples of the present invention. Further literature concerning any one of the materials, methods, uses and compounds to be employed in accordance with the present invention may be retrieved from public libraries and databases, using for example electronic devices. For example the public database "Medline" may be utilized, which is hosted by the National Center for Biotechnology Information and/or the National Library of Medicine at the National Institutes of Health. Further databases and web addresses, such as those of the European Bioinformatics Institute (EBI), which is part of the European Molecular Biology Laboratory (EMBL) are known to the person skilled in the art and can also be obtained using internet search engines. An overview of patent information in biotechnology and a survey of relevant sources of patent information useful for retrospective searching and for current awareness is given in Berks, TIBTECH 12 (1994), 352-364.

The above disclosure generally describes the present invention. Several documents are cited throughout the text of this specification. Full bibliographic citations may be found at the end of the specification immediately preceding the claims. The contents of all cited references (including literature references, issued patents, published patent applications as cited throughout this application and manufacturer's specifications, instructions, etc) are hereby expressly incorporated by reference; however, there is no admission that any document cited is indeed prior art as to the present invention.

The above disclosure generally describes the present invention. A more complete understanding can be obtained by reference to the following specific examples which are provided herein for purposes of illustration only and are not intended to limit the scope of the invention.

### EXAMPLES

The examples which follow further illustrate the invention, but should not be construed to limit the scope of the invention in any way. Detailed descriptions of conventional methods, such as those employed herein can be found in the cited literature; see also "The Merck Manual of Diagnosis and Therapy" Seventeenth Ed. ed. by Beers and Berkow (Merck & Co., Inc. 2003).

The practice of the present invention will employ, unless otherwise indicated, conventional techniques of cell biology, cell culture, molecular biology, transgenic biology, microbiology, recombinant DNA, and immunology, which are within the skill of the art.
Methods in molecular genetics and genetic engineering are described generally in the current editions of Molecular Cloning: A Laboratory Manual, (Sambrook et al., (1989) Molecular Cloning: A Laboratory Manual, 2nd ed., Cold Spring Harbor Laboratory Press); DNA Cloning, Volumes I and II (Glover ed., 1985); Oligonucleotide Synthesis (Gait ed., 1984); Nucleic Acid Hybridization (Hames and Higgins eds. 1984); Transcription And Translation (Hames and Higgins eds. 1984); Culture Of Animal Cells (Freshney and Alan, Liss, Inc., 1987); Gene Transfer Vectors for Mammalian Cells (Miller and Calos, eds.); Current Protocols in Molecular Biology and Short Protocols in Molecular Biology, 3rd Edition (Ausubel et al., eds.); and Recombinant DNA Methodology (Wu, ed., Academic Press). Gene Transfer Vectors For Mammalian Cells (Miller and Calos, eds., 1987, Cold Spring Harbor Laboratory); Methods In Enzymology, Vols. 154 and 155 (Wu et al., eds.); Immobilized Cells And Enzymes (IRL Press, 1986); Perbal, A Practical Guide To Molecular Cloning (1984); the treatise, Methods In Enzymology (Academic Press, Inc., N.Y.); Immunochemical Methods In Cell And Molecular Biology (Mayer and Walker, eds., Academic Press, London, 1987); Handbook Of Experimental Immunology, Volumes I-IV (Weir and Blackwell, eds., 1986). Reagents, cloning vectors, and kits for genetic manipulation referred to in this disclosure are available from commercial vendors such as BioRad, Stratagene, Invitrogen, and Clontech. General techniques in cell culture and media collection are outlined in Large Scale Mammalian Cell Culture (Hu et al., Curr. Opin. Biotechnol. 8 (1997), 148); Serum-free Media (Kitano, Biotechnology 17 (1991), 73); Large Scale Mammalian Cell Culture (Curr. Opin. Biotechnol. 2 (1991), 375); and Suspension Culture of Mammalian Cells (Birch et al., Bioprocess Technol. 19 (1990), 251); Extracting information from cDNA arrays, Herzel et al., CHAOS 11, (2001), 98-107.

### Example 1: Detection of interaction between JNK and TI-JIP

JNKs (c-jun N-terminal kinase) are important regulators of physiological and pathological processes. This subfamily of MAPKs (mitogen activated protein kinases) is involved in the development of various diseases including cancer, neurodegenerative diseases, cardiovascular diseases, inflammatory diseases and diabetes. In response to membrane receptor activation the kinases can phosphorylate serin, threonine, or tyrosine residues of different substrates and thereby transfer signals from the cell surface to the nucleus. JNKs themselves get activated by the phosphorylation at threonine 183 and tyrosine 185. Analysis of interaction of JNK1 (NM 002750, Aa 1-351) with peptides is of major interest for the development of peptide-derived specific inhibitors.

This example demonstrates that the present invention can be used for the detection of molecular interactions between JNK and selected peptides. Therefore, "bait" proteins were cloned into an appropriate vector for expression of heterologous proteins in yeast (pADRS RRSmut, modified from pRS vector Sikorski and Hieter, Genetics 122 (1989), 19-27) as a fusion protein with constitutive active human Ras (hRasΔ61) which is devoid of its membrane localization signal (ΔCAAX). Polymerase chain reaction (PCR) was used to generate different JNK fragments JNK APF (T¹⁸³ -A, Y¹⁸⁵-P) JNK K55A (K⁵⁵-A) and JNKwt. Following HindIII and SmaI restriction digests fragments were cloned into restriction sites to express amino terminal Ras fusion proteins. JNK APF is a dominant negative mutant with mutations in the phosphorylation sites. JNK K55A is a functional mutant which is not able to bind ATP. Interactions with the peptide JIP20 GPGDTYRPKRPTTLNLFPQVPRSG (SEQ ID NO: 1) were tested for the different JNKs. Peptides with similar amino acid sequences have already been shown to interact with JNK using alternative experimental methods; see for example Barr et al., J. Mol. Biol. 277 (13) (2002), 10987-10997, Barr et al., J. Mol. Biol. 279 (41) (2004),42178-42189).

The second plasmid (see Fig. 4), which contains the URA3 gene was constructed from the pYes2 plasmid (Invitrogen) by insertion of a nucleic acid, encoding a Src myristoylation signal ("m") for membrane localization and a thioredoxin (trx) expressing cDNA (M26133) to express peptides as loop in the RsrII site or as a linear peptide at the C-terminus. Thioredoxin presents JIP20 as loop by cloning it into the RsrII site of thioredoxin. The fragment encoding JIP20 was produced by annealing of primer JIP20fRSRII 5' gtc cgg ggg aca cgt acc ggc cca agc ggc cca cca cgc tca acc tct ttc cgc agg tgc cgc gga gcg 3' (SEQ ID NO: 2) and primer JIP20rRSRII 5' gac cgc tcc gcg gca cct gcg gaa aga ggt tga gcg tgg tgg gcc gct tgg gcc ggt acg tgt ccc ccg 3' (SEQ ID NO: 3). A restriction digest and cloning into the RsrII of thioredoxin was followed to allow a presentation of JIP20 as a loop.

The expression of the whole fusion protein is under the control of a GAL1 inducible promoter. Therefore, expression was induced by the addition of galactose to the medium (3% galactose and 2% raffinose). Glucose represses the activity of the GAL1 promoter and is therefore used in control plates to test the dependency of growth on the presence of the membrane localized peptide (specificity test); see Fig. 2.

Cdc25-2 yeast cells (MATα, ura3, lys2, Leu2, trpl cdc25-2, his3Δ200, ade 100, GAL+) were transformed with JNK constructs (wt, APF, K55A), empty vector (pADRS) and the pYesm-trx JIP20 construct (GAL1-myr-thioredoxin-JIP20aa/loop) by a modified high efficient transformation method according to Schiestl and Gietz, Curr. Genet. 16 (1989), 339-346. At a restrictive temperature of 30-37°C the cdc 25-2 yeast mutant does not express a functional active endogenous Ras-protein. Protein-peptide interaction results in activation of the Ras-pathway by recruitment of the hRas-fusion protein to the plasma membrane. Under these conditions growth of yeast cells can be observed at the restrictive temperature of 37°C.

Cdc25-2 cells were plated onto glucose as well as on galactose containing -Leu-Ura selection media and growth was analyzed depending on the expression of JIP20. Plates were incubated at 37°C and growth was observed after 5 days. As shown in Fig. 2a left part, cells expressing JNKwt, JNK APF, or JNKK55A and JIP20 stabilized in the loop grew at the restrictive temperature of 37°C. When expression of JIP20 was prevented by addition of glucose medium cells did not growth; see Fig. 2, right part.

In a control experiment cells were co-transformed with the same JNK1 constructs, but with a different Thioredoxin-JIP20 construct, lacking JIP (GAL1-myr-thioredoxin). The controls show that there is no cell growth in the absence of JIP20, demonstrating its dependency on the expression of JIP20 but not on the expression of thioredoxin alone; see Fig. 2b.

### Example 2: Isolation of JNK binding peptides from libraries

This example demonstrates that the invention is also useful for the identification of binding sequences from peptide populations. In the following example a JNK (c-Jun N-terminal kinase) binding peptide is isolated from a sequence population containing random as well as predefined peptides.

The "bait plasmid" (pADRS-JNKAPF) was constructed as already described in Example 1. Plasmids (pYESm-thioredoxin) that express defined JNK binding peptides either as loop inserted in the RsrII site of thioredoxin (as for JIP 20, Example 1) or as linear peptides at the C-terminus of thioredoxin (JIP 15 aa: TYRPKRPTTLNLFPQ; SEQ ID NO: 4) and control peptides (JIP 15 rev: QPFLNLTTPRKPRYT; SEQ ID NO: 5 and JIP 15 scr: LRFQPYPKNLTPTRT; SEQ ID NO: 6) were added in defined amounts (10 ng, 100 ng) to a random DNA sequence population from Hela cells (2 µg) which is expressed from the pYesm (described in Example 1). JIP 15 and control peptide sequences were generated by primer annealing and inserted into EcoRI/XhoI site in frame to the c-terminus of thioredoxin.

In comparison to the defined peptides, which are presented by a membrane localized thioredoxin, sequences from the random library also have a myristoylation signal but are not presented by the carrier. The amount of plasmid DNA expressing the peptides JIP 15, JIP20, JIPrev and JIPscr varied in different approaches between 10 ng and 100 ng. The DNA mixture was co-transformed into the yeast strain cdc25-2 with 300 ng pADRS JNK APF as "bait" plasmid according to the conventional transfection method as already described in Example 1. To select for "bait" and library plasmid the cells were plated on -Leu-Ura Glucose selection plate. After transferring the cells to galactose containing -Leu-Ura plate and incubation for 3-5 days at 37°C the growth selection occurs for clones expressing JNK1 APF binding sequences from the library. Preliminary positive clones were picked and incubated in liquid - leu-ura glucose. After two days of incubation a specificity test was done, where growth is analyzed at 37°C dependent on the expression of the library peptide (under galactose conditions). The number of positive clones varied between 66 up to 90 (for 10 ng/2 µg Hela DNA) and 368 up to 452 (for 100 ng/2 µg Hela) for JIP20 and JIP15 peptide. In all approaches the same amount (10 ng, 100 ng) of control peptides was added. To further evaluate the clones PCR was performed. Exemplary results from colony PCR of clones from a JIP20 screening are shown in Fig. 3.

A first colony PCR was done to analyze the existence of thioredoxin with the following primer: P 1 trxASmaIforw 5' - ccc ccc ggg atg agc gat aaa att att cac c-3' (SEQ ID NO: 7) and P2 trxAEcoRIrev 5'-ttt tga att ccc gcc agg tta gcg tcg ag -3' (SEQ ID NO: 8) resulting in a 416 bp fragment and in a second colony PCR the existence of JIP 20 was analyzed by using primer P3 trxASmaIforw 5'- ccc ccc ggg atg agc gat aaa att att cac c -3' (SEQ ID NO: 9) and P4 JipXho 5' tcg agt cac tgc gga aag agg ttg agc gtg gtg ggc -3' (SEQ ID NO: 10) resulting in a 162 bp fragment. As shown in Fig. 3, yeast clones (B9, C9, D9, A4, A5, A1 and C3) were positive for the existence of the 416 bp thioredoxin fragment as well as for the existence of the 162 bp JIP 20 fragment. Clones B3 and F6 were also considered as positive after the growth analysis, however, a positive PCR result was not obtained. Those clones could express JNK-interacting proteins from the Hela library.

In summary, the experiments performed in accordance with the present invention demonstrate the power of the method and assay of the present invention for identifying and obtaining peptides such as peptide-ligands capable of interacting with a target protein and thus is expected to prove useful for the identification of yet unknown binding partners as well as in drug development.

## Claims

1. Method of identifying a protein-peptide interaction or isolating a partner of a protein-peptide interaction, comprising:
(a) providing within a host cell, wherein the host cell preferably lacks an active endogenous effector;
(i) a hybrid molecule, preferably fusion protein, comprising a target protein and an effector;
(ii) a peptide or a population of peptides, wherein the peptide is linked to and preferably embedded within a carrier protein, said carrier protein being linked to a cell compartment localization domain, preferably membrane localization domain; and
(b) detection of a signal identifying the protein-peptide interaction of the hybrid molecule and the peptide; and optionally
(c) isolating a partner of the protein-peptide interaction based on its ability to alter the signal when present.

2. The method of claim, wherein the host cell is a yeast cell, preferably a *saccharomyces cerevisiae* cdc25-2 cell.

3. The method of claim 1 or 2, wherein the effector is a guanine nucleotide exchange factor (GEF), Sos-protein or Ras-protein.

4. The method of claim 3, wherein the effector is a mutated Ras-protein such that it cannot localize to the cell membrane and does not require an exchange factor or the detectable signal is to the activation of the Ras-protein and the effector is a guanine nucleotide exchange factor (GEF).

5. The method of any one claims 1 to 4, wherein the carrier protein is thioredoxin or a thioredoxin-like molecule.

6. The method of any one of claims 1 to 5, wherein the membrane localization domain is a plasma membrane localization domain, preferably a myristoylation signal.

7. Method of detecting and/or isolating an interacting peptide in a population of peptides comprising:
(a) providing a hybrid molecule, comprising a target protein and an effector within a host cell;
(b) introducing a test peptide or a population of test peptides into the host cell, wherein the peptide is linked to a carrier protein, said carrier protein being linked to a cell compartment localisation domain;
(c) detection of a signal identifying the protein-peptide interaction of the hybrid molecule and the peptide; and optionally
(d) isolating a peptide, identified as a binding partner based on its ability to alter the signal when present.

8. Method for isolating a protein comprising:
(a) providing a host cell comprising a test protein or a population of test proteins and an effector, or introducing the effector;
(b) introducing the linkage of the effector to the test protein or population of test proteins;
(c) providing or introducing a peptide or population of peptides within/into the host cell, wherein the peptide is linked to a carrier protein, said carrier protein being linked to a cell compartment localisation domain;
(d) detection of a signal identifying the protein-peptide interaction; and
(e) isolating the protein identified as a binding partner based on its ability to alter the signal when present.

9. Use of a method of any one of claims 1 to 8 for identifying and optionally isolating an antagonist or agonist molecule and/or for lead compound and drug development.

10. A kit or assay system for performing the method of any one of claims 1 to 8 or the use of claim 9, said kit or assay system comprising a component as featured in any one of claims 1 to 8 and/or reagents, automation tools, storage ware, handling devices, liquid handling robots, monitoring arrangement or the like.
